# EUROPEAN PATENT APPLICATION

(11) **EP 4 324 500 A1**
(43) Date of publication of application: **21.02.2024**
(21) Application number: 21936742.2
(22) Date of filing: 19.10.2021
(51) Int. Cl.: A61M 5/178, A61M 5/24, A61M 5/31

(54) **MULTI-DOSE ADJUSTABLE PRE-CHARGE INJECTION PEN**

(30) Priority: 16.04.2021 CN 202110411143; 16.04.2021 CN 202120784042 U
(71) Applicant: Shandong Wego Prefills Pharmaceutical Packaging Co., Ltd., Weihai, Shandong 264210 (CN)
(72) Inventor: NI, Shili, Weihai, Shandong 264210 (CN); SUN, Xiaofeng, Weihai, Shandong 264210 (CN); XU, Qianhe, Weihai, Shandong 264210 (CN); LI, Wenzheng, Weihai, Shandong 264210 (CN); LI, Zhifei, Weihai, Shandong 264210 (CN)
(74) Representative: Ungria López, Javier
(86) International application number: PCT/CN2021/124591
(87) International publication number: WO 2022/217875

(57) **Abstract**

A multi-dose adjustable pre-charge injection pen, comprising a pen body, an injection push rod (13), a push rod sleeve (7), a clutch hub (6), an end limiting member (8), and a dose hub (14). The injection push rod (13) is in threaded connection with the pen body; the push rod sleeve (7) and the injection push rod (13) are in sliding fit with each other in an injection direction and can transmit torque in a screwing-in direction of the injection push rod (13); the push rod sleeve (7) and the pen body are connected by means of a first set of ratchet pawl mechanism; the clutch hub (6) and the push rod sleeve (7) are in sliding fit with each other in the injection direction; the dose hub (14) is rotatably connected to the pen body by means of threads; rotation between the clutch hub (6) and the dose hub (14) is limited by a fastening structure capable of being disengaged from the injection direction; the end limiting member (8) and the dose hub (14) are supported by a supporting foot (83) capable of elastically deforming in the injection direction; the dose hub (14) can be rotated to push the fastening structure to be disengaged before the supporting foot (83) elastically deforms, thereby effectively solving the problem that an existing injection pen is inconvenient to operate and the dose adjustment effect is poor.

## Description

This application claims the benefit of priorities to the following two Chinese patent applications, both of which are incorporated herein by reference in their entireties:
1) Chinese patent application No. 202110411143.1, titled "MULTI-DOSE ADJUSTABLE PRE-FILLED INJECTION PEN", filed on April 16, 2021 with the China National Intellectual Property Administration, and
2) Chinese Patent Application No. 202120784042.4, titled "MULTI-DOSE ADJUSTABLE PRE-FILLED INJECTION PEN", filed on April 16, 2021 with the China National Intellectual Property Administration.

### FIELD

The present application relates to the technical field of injection devices and in particular to a multi-dose adjustable pre-filled injection pen.

### BACKGROUND

The conventional injection pen products have a complex structure and a large number of parts, and have low reliability due to excessive processing procedures. For some of the products, parts thereof are bonded by using glue during processing, which has biological risks. Lots of the parts of the products are made of metal, resulting in an increase in the cost of the products and a poor use effect.

In summary, how to effectively solve existing problems of inconvenient operation and poor dose adjustment for the injection pen is a major concern of those skilled in the art at present.

### SUMMARY

In view of the above, it is an object of the present application to provide a multi-dose adjustable pre-filled injection pen which can effectively solve existing problems of inconvenient operation and poor dose adjustment for an injection pen.

In order to achieve the above object, the following technical solutions are provided according to the present application.

A multi-dose adjustable pre-filled injection pen includes a pen body, an injection push rod, a push rod sleeve, a clutch hub, an end limiting member and a dose hub. The injection push rod is connected to the pen body through threads having an axis extending in an injection direction. The push rod sleeve is in sliding fit with the injection push rod in the injection direction and is configured to transmit a torque in a direction of screwing-in of the injection push rod. The push rod sleeve is connected to the pen body through a first set of ratchet pawl mechanisms, which precludes the push rod sleeve from rotating, relative to the pen body, in a direction of screwing-out of the injection push rod. The clutch hub is in sliding fit with the push rod sleeve in the injection direction, and relative rotation between the clutch hub and the push rod sleeve is precluded. The dose hub is rotatably connected to the pen body through threads, and is configured to be pushed to screw in by driving axially. A relative rotation between the clutch hub and the dose hub is precluded by a clutch structure which disengages in the injection direction. A support foot elastically deformable in the injection direction is braced between the end limiting member and the dose hub. The clutch structure is prevented from disengaging by the support foot, which results from the deformation of the support foot due to the fact that the end limiting member is subjected to a thrust in the injection direction. The clutch structure is configured to be pushed to disengage by rotation of the dose hub before the support foot deforms elastically.

In the use of the multi-dose adjustable pre-filled injection pen, a cartridge is fixed to the pen body, and the injection push rod is then placed against a rubber stopper of the cartridge, wherein the dose hub is in an initial position relative to the pen body. Then, the end limiting member is pushed in the injection direction to elastically deform the support foot and to prevent the clutch structure from disengaging, wherein the end limiting member transfers the thrust in the injection direction to the dose hub, and the dose hub then screws in. Since the clutch structure is in engagement, the dose hub drives the clutch hub to rotate, to drive the push rod sleeve and the injection push rod to rotate in sequence for injection. After the injection is completed for the first time, the end limiting member is released and reset, and the dose hub returns to the initial position to allow re-adjustment of the dose for a next injection. In a case that the injection pen is adjusted to increase the dose, the dose hub may be operated to screw out relative to the pen body. In the process of screwing out, the dose hub cannot drive the clutch hub to rotate due to the absence of limitation by the end limiting member, but drives the clutch hub to slide in a direction opposite to the injection direction, while the injection push rod and the push rod sleeve are static relative to the pen body. In a case that the injection pen is adjusted to decrease the dose, the dose hub is operated to screw in, and the clutch hub does not rotate because the end limiting member is not subjected to a force in the injection direction. In the multi-dose adjustable pre-filled injection pen, the end limiting member is provided to control the rotational connection between the dose hub and the clutch hub through the flexible support foot. With the above arrangement, the injection can be performed by directly pressing the end limiting member in a case that the injection is required, and in a case that the dose is adjusted by rotation, it is only required to rotate the dose hub. The operation is very convenient and simple, and the operation of adjusting the dose is simple, the overall structure is very simple, few parts are included and easy to be assembled, and it is not necessary to use metal springs and other structures, thereby realizing a lower overall cost. In summary, with the multi-dose adjustable pre-filled injection pen, existing problems of inconvenient operation and poor dose adjustment of the injection pen can be effectively solved.

In an embodiment, the multi-dose adjustable pre-filled injection pen further includes a dose limiting nut, where the dose limiting nut is connected to the clutch hub by threads and in sliding fit with the dose hub in the injection direction, relative rotation between the dose limiting nut and the dose hub is precluded, and the dose limiting nut moves along an axial direction of the clutch hub when the dose hub is rotated relative to the clutch hub.

In an embodiment, a pawl is arranged at a tip of the support foot, and a ratchet fitting with the pawl of the support foot is arranged at an end of the dose hub, to preclude relative rotation in the direction of screwing-in; the end limiting member is provided with a limiting flange, and the dose hub is correspondingly provided with a limiting groove enclosing the limiting flange, to limit a range of movement of the limiting flange in the injection direction.

In an embodiment, a protruding disc is arranged at a rear end of the clutch hub, multiple arc-shaped protrusions circumferentially distributed evenly are arranged at a front side of the protruding disc, an annular shoulder is arranged inside a rear end of the dose hub, arc-shaped tooth grooves circumferentially distributed and fitting with the arc-shaped protrusions are arranged at a rear side of the annular shoulder, and the rear end of the clutch hub and the end limiting member are connected through a second set of ratchet pawl mechanisms.

In an embodiment, the multi-dose adjustable pre-filled injection pen further includes an end button, where a circumference of the end button is fitted into the end limiting member, a force transmission cylinder extending in the injection direction is arranged in the middle of the end button, and a front end of the force transmission cylinder abuts against the end limiting member and is spherical.

In an embodiment, the pen body includes a body barrel and a push rod nut fitted into the body barrel, where the push rod nut is connected to the injection push rod by threads and connected to the push rod sleeve through the first set of ratchet pawl mechanisms.

In an embodiment, the injection push rod, the push rod sleeve, the clutch hub, the dose limiting nut, the dose hub and the body barrel are arranged in sequence with a latter sleeved on a former.

In an embodiment, a window is provided on the pen body, and spirally distributed scales are provided at an outer side of the dose hub, where the scales pass through the window in sequence when the dose hub is screwed in.

In an embodiment, the multi-dose adjustable pre-filled injection pen further includes a vial holder clamped at a front end of the body barrel, where a vial ring is arranged at a rear end of the vial holder, the vial ring is configured to abut against a vial in the vial holder at a front end and abut against the pen body at a rear end, a rotatable push rod knob configured to abut against a vial rubber stopper is provided at a front end of the injection push rod, and a threaded post is provided at a front end of the vial holder for attaching a needle.

In an embodiment, the multi-dose adjustable pre-filled injection pen further includes a pen cap, where a rear end of the pen cap is snap-fitted to the vial holder.

### BRIEF DESCRIPTION OF THE DRAWINGS

In order to more clearly illustrate technical solutions in embodiments of the present application or in the conventional technology, the drawing referred to for describing the embodiments or the conventional technology will be briefly described hereinafter. Apparently, the drawings in the following description are only some examples of the present application, and for those skilled in the art, other drawings may be obtained based on the provided drawings without any creative efforts.
FIG. 1 is a schematic cross-sectional structural view of a multi-dose adjustable pre-filled injection pen according to an embodiment of the present application;
FIG. 2 is a schematic exploded structural view of the multi-dose adjustable pre-filled injection pen according to an embodiment of the present application;
FIG. 3 is a schematic external structural view of the multi-dose adjustable pre-filled injection pen according to an embodiment of the present application, where a pen cap is removed;
FIG. 4 is a schematic structural view of the multi-dose adjustable pre-filled injection pen according to an embodiment of the present application, where a dose hub is screwed out;
FIG. 5 is a schematic cross-sectional structural view of the multi-dose adjustable pre-filled injection pen according to an embodiment of the present application, where the dose hub is screwed out;
FIG. 6 is a schematic structural view of a push rod nut according to an embodiment of the present application;
FIG. 7 is a schematic structural view of a dose limiting nut according to an embodiment of the present application;
FIG. 8 is a schematic structural view of a clutch hub according to an embodiment of the present application;
FIG. 9 is a schematic structural view of a push rod sleeve according to an embodiment of the present application;
FIG. 10 is a schematic structural view of an end limiting member according to an embodiment of the present application;
FIG. 11 is a schematic structural view of a vial holder according to an embodiment of the present application;
FIG. 12 is a schematic structural view of the dose hub according to an embodiment of the present application;
FIG. 13 is a schematic internal structural view of a body barrel opening according to an embodiment of the present application;
FIG. 14 is a schematic structural view of a rear end of the multi-dose adjustable pre-filled injection pen according to an embodiment of the present application;
FIG. 15 is a schematic exploded structural view of the rear end of the multi-dose adjustable pre-filled injection pen according to an embodiment of the present application;
FIG. 16 is a schematic cross-sectional structural view of the rear end of the multi-dose adjustable pre-filled injection pen according to an embodiment of the present application;
FIG. 17 is a schematic mounting view of the clutch hub and the dose hub according to an embodiment of the present application;
FIG. 18 is a schematic cross-sectional mounting structural view of the rear end of the multi-dose adjustable pre-filled injection pen according to an embodiment of the present application; and
FIG. 19 is a schematic mounting structural view of the dose limiting nut and the dose hub according to an embodiment of the present application.

The reference numerals in the drawings are listed as follows.

| | | |
|---|---|---|
| needle 1, | cartridge 2, | rubber stopper 3, |
| push rod nut 4, | dose limiting nut 5, | clutch hub 6, |
| push rod sleeve 7, | end limiting member 8, | pen cap 9, |
| vial holder 10, | push rod knob 11, | vial ring 12, |
| injection push rod 13, | dose hub 14, | body barrel 15, |
| injection button 16, | threaded hole 41, | limiting ratchet 42, |
| third groove 43, | second groove 51, | limiting female thread 52, |
| first rib 61, | arc-shaped protrusion 62, | |
| transmission pawl 63, | limiting male thread 64, | |
| cut plane 71, | limiting pawl 72, | first groove 73, |
| hook 74, | annular outward protrusion 81, | |
| limiting flange 82, | support foot 83, | transmission ratchet 84, |
| notch 101, | mounting protrusion 102, | |
| limiting connection structure 103, | | |
| threaded post 104, | support ratchet 141, | |
| arc-shaped tooth groove 142, | | |
| zero mark 143, | radial spline 144, | adjusting male thread 145, |
| limiting groove 146, | second rib 147, | adjusting female thread 151, |
| window 152, | reading mark 153, | third protrusion 154, |
| limiting protrusion 155, | annular mounting groove 156, | |
| force transmission cylinder 161, | | annular inward protrusion 162 |

### DETAILED DESCRIPTION OF THE EMBODIMENTS

A multi-dose adjustable pre-filled injection pen is provided according to an embodiment of the present application to effectively solve existing problems of inconvenient operation and poor dose adjustment for an injection pen.

Technical solutions in the embodiments of the present application will be described clearly and completely hereinafter in conjunction with the drawings in the embodiments of the present application. Apparently, the embodiments described in the following are only some embodiments of the present application, rather than all embodiments. Any other embodiments, obtained by those skilled in the art on the basis of the embodiments in the present application without any creative efforts, fall into the scope of protection of the present application.

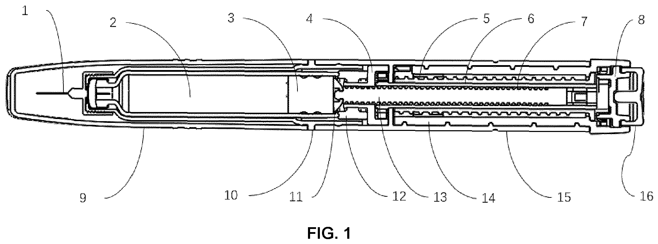

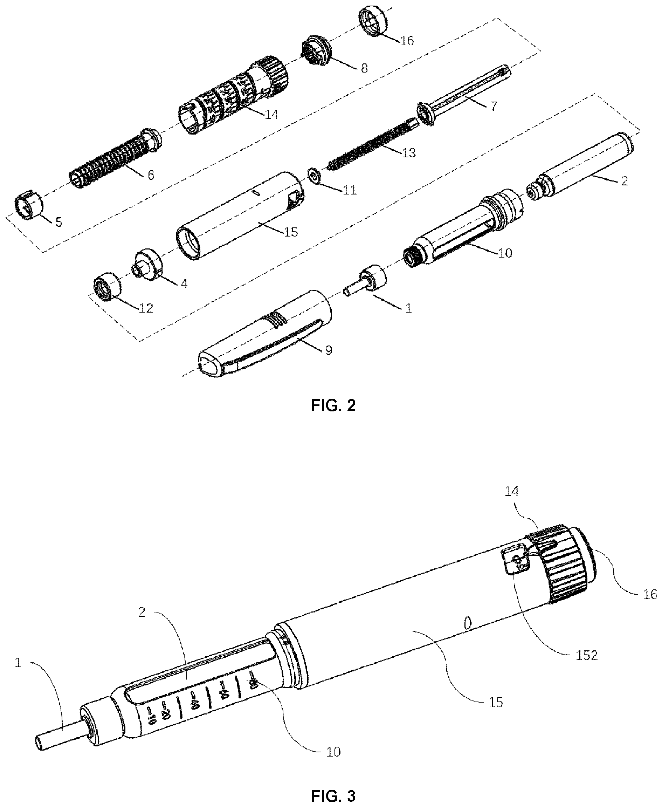

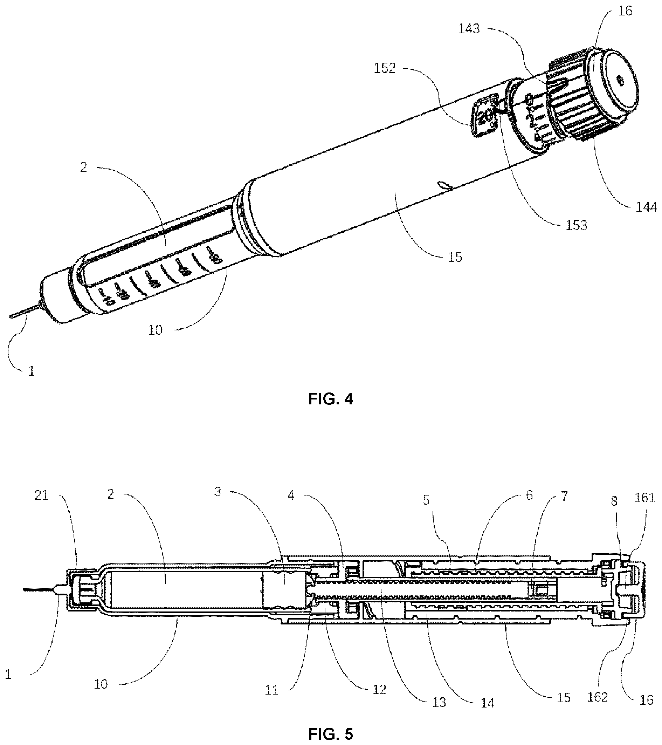

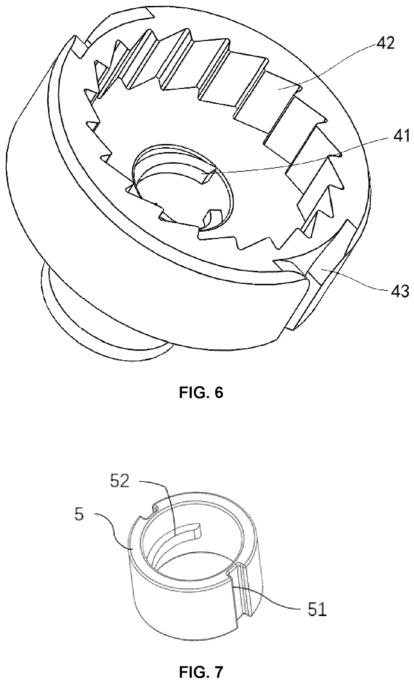

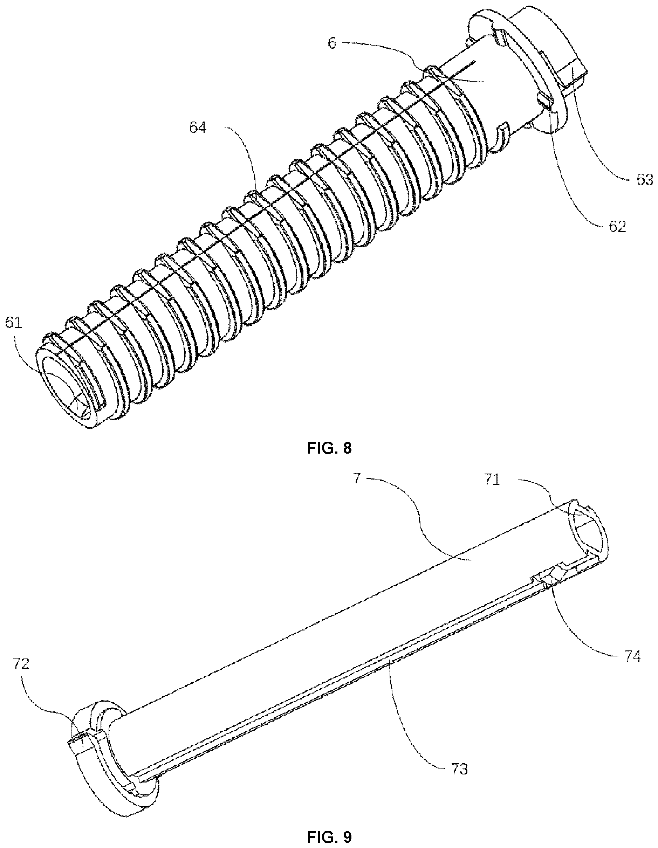

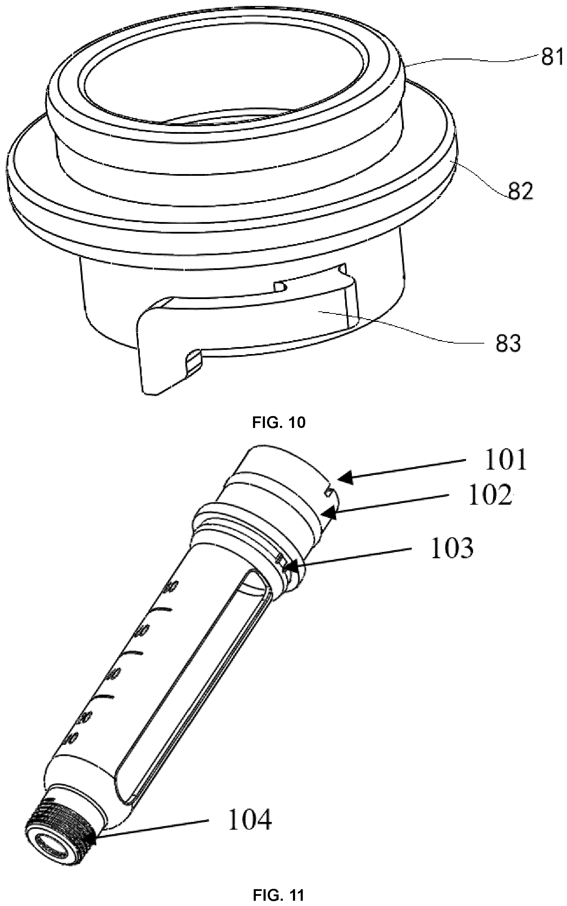

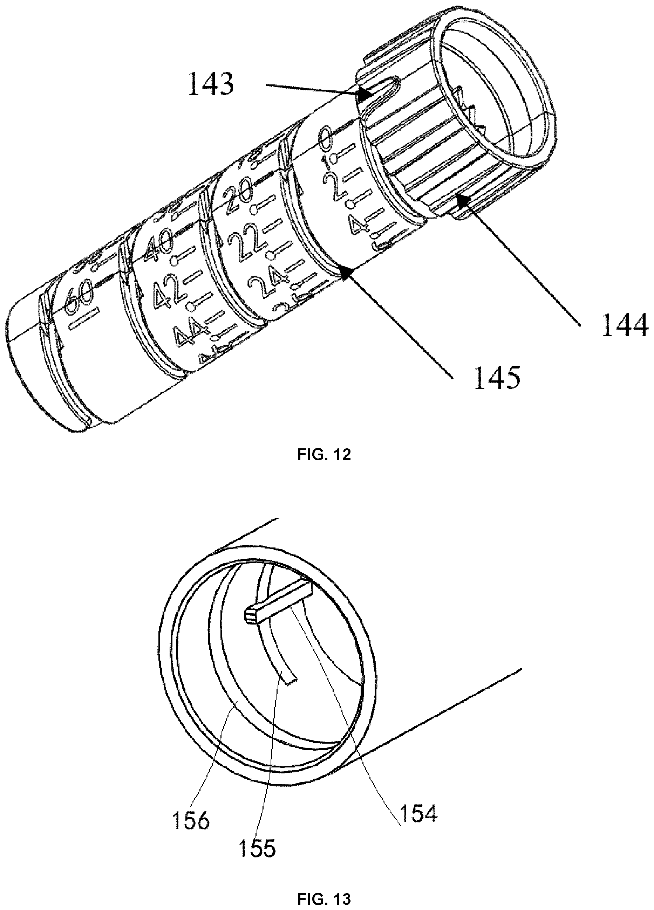

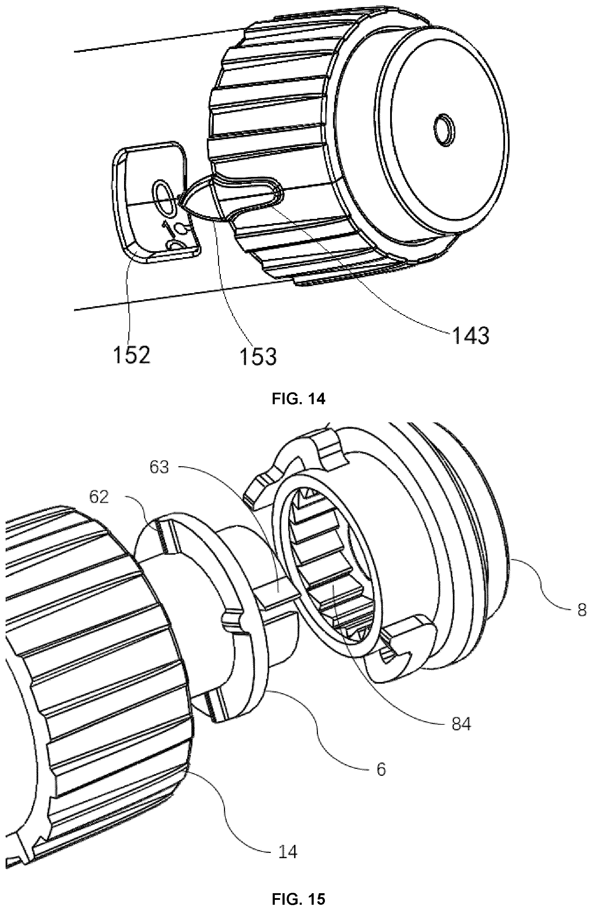

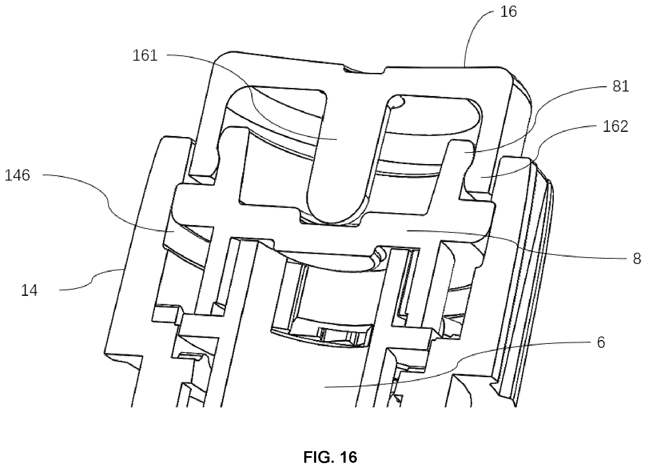

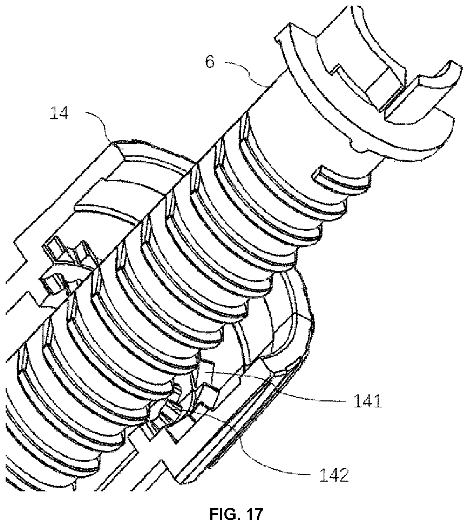

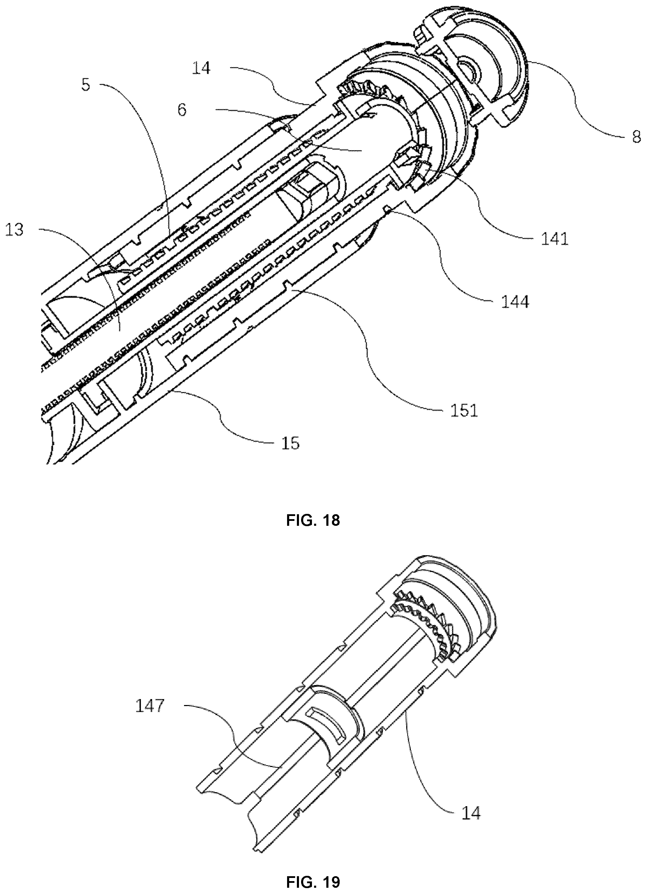

Referring to FIGS. 1 to 19, FIG. 1 is a schematic cross-sectional structural view of a multi-dose adjustable pre-filled injection pen according to an embodiment of the present application; FIG. 2 is a schematic exploded structural view of the multi-dose adjustable pre-filled injection pen according to an embodiment of the present application; FIG. 3 is a schematic external structural view of the multi-dose adjustable pre-filled injection pen according to an embodiment of the present application, where a pen cap is removed; FIG. 4 is a schematic structural view of the multi-dose adjustable pre-filled injection pen according to an embodiment of the present application, where a dose hub is screwed out; FIG. 5 is a schematic cross-sectional structural view of the multi-dose adjustable pre-filled injection pen according to an embodiment of the present application, where the dose hub is screwed out; FIG. 6 is a schematic structural view of a push rod nut according to an embodiment of the present application; FIG. 7 is a schematic structural view of a dose limiting nut according to an embodiment of the present application; FIG. 8 is a schematic structural view of a clutch hub according to an embodiment of the present application; FIG. 9 is a schematic structural view of a push rod sleeve according to an embodiment of the present application; FIG. 10 is a schematic structural view of a vial holder according to an embodiment of the present application; FIG. 11 is a schematic structural view of a vial holder according to an embodiment of the present application; FIG. 12 is a schematic structural view of the dose hub according to an embodiment of the present application; FIG. 13 is a schematic internal structural view of a body barrel opening according to an embodiment of the present application; FIG. 14 is a schematic structural view of a rear end of the multi-dose adjustable pre-filled injection pen according to an embodiment of the present application; FIG. 15 is a schematic exploded structural view of the rear end of the multi-dose adjustable pre-filled injection pen according to an embodiment of the present application; FIG. 16 is a schematic cross-sectional structural view of the rear end of the multi-dose adjustable pre-filled injection pen according to an embodiment of the present application; FIG. 17 is a schematic mounting view of the clutch hub and the dose hub according to an embodiment of the present application; FIG. 18 is a schematic cross-sectional mounting structural view of the rear end of the multi-dose adjustable pre-filled injection pen according to an embodiment of the present application; and FIG. 19 is a schematic mounting structural view of the dose limiting nut and the dose hub according to an embodiment of the present application.

A multi-dose adjustable pre-filled injection pen is provided according to an embodiment of the present application. As shown in FIGS. 1 to 3, the multi-dose adjustable pre-filled injection pen mainly includes a pen body, an injection push rod 13, a push rod sleeve 7, a clutch hub 6, an end limiting member 8 and a dose hub 14. A vial holder 10 for placing a cartridge 2, a pen cap 9 sleeved on the pen body, and the like may be further included.

The injection push rod 13 is connected to the pen body through threads having an axis extending in an injection direction, so that when the injection push rod 13 is rotated in the direction of screwing in, the injection push rod 13 is also moved axially relative to the pen body. Further, the cartridge 2 and the pen body are fixed relatively to each other generally through the vial holder 10, or through snap-fitting, threaded connection and other connection manners. The injection push rod 13 is capable of pushing a rubber stopper 3 inside the cartridge 2 to move in the injection direction, i.e. to form an injection.

The push rod sleeve 7 is in sliding fit with the injection push rod 13 in the injection direction and is configured to transmit a torque in the direction of screwing-in of the injection push rod. In an embodiment, the injection push rod 13 is provided with male threads. The injection push rod 13 may be cut flat on one side or two opposite sides, and the push rod sleeve 7 is sleeved outside the injection push rod 13 with cross-sections corresponding to each other. That is, cut planes 71, e.g. in a flattened shape, are correspondingly provided, in order to limit the rotation of the internal injection push rod 13. The injection push rod 13 is slidable in the injection direction inside the push rod sleeve 7 to achieve a sliding connection. Alternatively, a groove extending axially is defined in the injection push rod 13, and a corresponding rib is provided at an inner side of the push rod sleeve 7, in order to preclude the relative rotation between the push rod sleeve 7 and the injection push rod 13 by the rid and the groove.

The push rod sleeve 7 is connected to the pen body through a first set of ratchet pawl mechanisms, which precludes the push rod sleeve 7 from rotating relative to the pen body in the direction of screwing out of the injection push rod 13. The clutch hub 6 is in sliding fit with the push rod sleeve 7 in the injection direction and relative rotation between the clutch hub 6 and the push rod sleeve 7 is precluded. Since the relative rotation between the push rod sleeve 7 and the injection push rod 13 is precluded, rotation of the injection push rod 13 in the direction of screwing out is also precluded, so that the push rod sleeve 7, the injection push rod 13 and the clutch hub 6 all can only rotate in the direction of screwing in of the injection push rod 13. When the injection push rod 13 is screwed in, the injection is thereby performed. When the injection push rod 13 is screwed in, the push rod sleeve 7 rotates accordingly but is fixed relative to the pen body in the injection direction, and the injection push rod 13 slides relative to the push rod sleeve 7 in the injection direction. The clutch hub 6 is in sliding fit with the push rod sleeve 7 in the injection direction and relative rotation therebetween is precluded. In an embodiment, the clutch hub 6 may be sleeved at an outer side of the push rod sleeve 7, and one of an outer side face of the push rod sleeve 7 and an inner side face of the clutch hub 6 is provided with a rib extending in the injection direction, and the other is provided with a groove that fits with the rib, to preclude the relative rotation. In an embodiment, two sides of the push rod sleeve 7 are each provided with a first groove 73, and two internal opposite sides of the clutch hub 6 are each provided with a corresponding first rib 61.

The dose hub 14 is rotatably connected to the pen body by threads, and the dose hub 14 is driven to be screwed in when being driven axially, i.e. the dose hub 14 is connected to the pen body preferably by non-self-locking threads. In order to ensure smoother rotation, a sliding friction at the threaded connection between the dose hub 14 and the pen body is required to be minimized, and lubricant may be added between the dose hub 14 and the pen body. Alternatively, the non-self-locking threads may be replaced with other structures which can convert the axial thrust partially into a torque thrust to drive the dose hub 14 to rotate relative to the pen body. In an embodiment, adjusting male threads 145 may be provided on the outer side of the dose hub 14, and adjusting female threads 151 may be provided on the inner side of the pen body.

The rotation between the clutch hub 6 and the dose hub 14 is precluded through a clutch structure that can be disengaged in the injection direction, and the dose hub 14 can provide support for the clutch hub 6 in a direction opposite to the injection direction while the clutch structure is engaged. That is, the clutch hub 6 is movable in the injection direction to allow the clutch structure to enter the engaged state. While the clutch structure is not disengaged, the relative rotation between the dose hub 14 and the clutch hub 6 is precluded, i.e., the rotation of the dose hub 14 drives the clutch hub 6 to rotate. While the clutch structure is disengaged, the dose hub 14 and the clutch hub 6 are rotatable freely relative to each other.

A support foot 83 elastically deformable in the injection direction is braced between the end limiting member 8 and the dose hub 14, so that when the end limiting member 8 is subjected to a thrust in the injection direction and the support foot 83 is thereby elastically deformed, the clutch structure is prevented from disengaging by the support foot 83, while before the support foot 83 is elastically deformed, the clutch structure is capable of being pushed to disengage by rotation of the dose hub 14, even if the clutch structure is in the engaged state. Simply rotating the dose hub 14, in the absence of limitation by the end limiting member 83, forces the clutch structure to disengage, so that the torque cannot be transmitted and the clutch hub 6 cannot be driven to rotate synchronously.

In the use of the multi-dose adjustable pre-filled injection pen, the cartridge 2 is fixed to the pen body, and the injection push rod 13 is then placed against the rubber stopper 3 of the cartridge 2, wherein the dose hub 14 is in an initial position relative to the pen body. Then, the end limiting member 8 is pushed in the injection direction to elastically deform the support foot 83 and to prevent the clutch structure from disengaging, wherein the end limiting member 8 transfers the thrust in the injection direction to the dose hub 14, and the dose hub 14 then screws in. Since the clutch structure is in engagement, the dose hub 14 drives the clutch hub 6 to rotate, to drive the push rod sleeve 7 and the injection push rod 13 to rotate in sequence for injection. After the injection is completed for the first time, the end limiting member 8 is released and reset, and the dose hub 14 returns to the initial position to allow re-adjustment of the dose for a next injection. Then, the dose hub 14 may be operated to screw out relative to the pen body. In the process of screwing out, the dose hub 14 cannot drive the clutch hub 6 to rotate due to the absence of limitation by the end limiting member 8, but drives the clutch hub 6 to slide in a direction opposite to the injection direction, while the injection push rod 13 and the push rod sleeve 7 remain static relative to the pen body until the dose hub 14 returns to the initial position relative to the pen body to be ready for the next injection. In the event of excessive rotation, the dose hub 14 is required to be screwed in, and the clutch hub 6 does not rotate because the end limiting member 8 is not subjected to a force in the injection direction. In the multi-dose adjustable pre-filled injection pen, the end limiting member 8 is provided to control the rotational connection between the dose hub 14 and the clutch hub 6 through the flexible support foot 83. With the above arrangement, the injection can be performed by directly pressing the end limiting member in a case that the injection is required, and in a case that the dose is adjusted by rotation, it is only required to rotate the dose hub 14. The operation is very convenient and simple, and the operation of adjusting the dose is simple, the overall structure is very simple, few parts are included and easy to be assembled, and it is not necessary to use metal springs and other structures, thereby realizing a lower overall cost. In summary, with the multi-dose adjustable pre-filled injection pen, existing problems of inconvenient operation and poor dose adjustment of the injection pen can be effectively solved.

Further, in order to avoid that the indicated dose exceeds the contained dose which is caused by excessive rotation of the dose hub 14, preferably, a dose limiting nut 5 is further included. In an embodiment, the dose limiting nut 5 is connected to the clutch hub 6 by threads, and is in sliding fit with the dose hub 14 in the injection direction and relative rotation therebetween is precluded, so that when the dose hub 14 is rotated relative to the clutch hub 6, the dose limiting nut 5 moves in an axial direction of the clutch hub 6, i.e. in the injection direction; and when the dose hub 14 reaches a predetermined angle of rotation relative to the clutch hub 6, the dose limiting nut 5 cannot further rotate relative to the clutch hub 6, and the dose hub 14 thereby cannot further rotate relative to the clutch hub 6 either. In an embodiment, the dose limiting nut 5 may include limiting female threads 52 and a second groove 51. Two second grooves 51 extending in the injection direction are respectively provided at two side of the dose limiting nut, and two second ribs 147 are respectively provided at two internal opposite sides of the dose hub 14 to limit the rotation of the dose hub 14 relative to the dose limiting nut 5 by means of the second grooves 51 and the second ribs 147. Correspondingly, limiting male threads 64 are provided at an outer side of the clutch hub 6 to mate with the limiting female threads 52. In an embodiment, a pitch of threads between the dose limiting nut 5 and the clutch hub 6 may be smaller than a pitch of threads between the dose hub 14 and the pen body, and the threads between the dose limiting nut 5 and the clutch hub 6 may be self-locking threads.

Further, in order to avoid rotation of the end limiting member 8 relative to the dose hub 14 during injection, it is preferred here that a tip of the support foot 83 is a pawl and a tip of the pawl is directed towards the injection direction. The support foot 83 is rod-shaped, wherein one end thereof is provided with the pawl which extends and protrudes downwardly to act as a support point, and the other end thereof is connected to a body of the end limiting member 8 to allow the support foot 83 to bend elastically to further change a relative distance between the pawl and the body of the end limiting member 8 in the injection direction.

Support ratchets 141 fitting with the pawl on the support foot 83 are provided at an end of the dose hub 14, to prevent relative rotation in the direction of screwing in of the dose hub 14, so that the dose hub 14 can drive the end limiting member 8 to rotate synchronously as the dose hub 14 screws in and provide support for the end limiting member 8 in a direction opposite to the injection direction, i.e. in a backward direction.

Further, in order to avoid the end limiting member 8 from wobbling freely, especially in the injection direction, it is preferred that the end limiting member 8 is provided with a limiting flange 82, and that the dose hub 14 is provided with a limiting groove 146 enclosing the limiting flange 82, in order to limit a range of movement of the limiting flange 82 in the injection direction and to limit the range of movement of the end limiting member 8 relative to the dose hub 14 in the injection direction. The support foot 83 on the end limiting member 8 is preferably in a pre-tightened state.

Further, in order to facilitate arranging the clutch structure, in an embodiment, a protruding disc may be arranged at a rear end of the clutch hub 6 and multiple arc-shaped protrusions 62 circumferentially distributed evenly are arranged at a front side of the protruding disc. Correspondingly, an annular shoulder is arranged inside the rear end of the dose hub 14 and arc-shaped tooth grooves 142 circumferentially distributed and fitting with the arc-shaped protrusions 62 are arranged at a rear end of the annular shoulder. In an embodiment, each of the arc-shaped protrusions 62 may be semi-cylindrical and have an axial direction consistent with the radial direction of the clutch hub 6, and the corresponding arc-shaped tooth groove 142 has a semi-circular cross-section, so that the clutch hub 6 can easily fluctuate in the injection direction during the rotation of the dose hub 14. Therefore, it is easy for the clutch structure to get into or out of the engaged state to avoid transmitting a torque.

Further, the rear end of the clutch hub 6 may be connected to the end limiting member 8 through a second set of ratchet pawl mechanisms, i.e. transmission pawls 63 arranged oppositely are arranged at the rear end of the clutch hub 6 and transmission ratchets 84 are arranged within the end limiting member 8, to enable the dose hub 14 to transmit a torque to the end limiting member 8 in the injection and enable the end limiting member 8 to transmit a torque to the clutch hub 6. During dose adjustment, the transmission pawls 63 are disconnected from the transmission ratchets 84 due to the support foot 83.

Further, in order to facilitate the application of a thrust in the injection direction to the end limiting member 8 in the injection, an end button 16 is preferably further included. A circumference of the end button 16 is fitted into the end limiting member 8, in order to avoid the end button 16 from disengaging from the end limiting member 8. In an embodiment, the end limiting member 8 is provided with an annular outward protrusion 81 annularly extending and the end button is correspondingly provided with an annular inward protrusion 162 inwardly protruding, wherein a front side of the annular outward protrusion 81 abuts against a rear side of the annular inward protrusion 162. Preferably, a force transmission cylinder 161 extending in the injection direction is arranged in the middle of the end button 16, and a front end of the force transmission cylinder 161 abuts against the end limiting member 8 and is spherical, to reduce the resistance to rotation.

In an embodiment, the end button 6 may be of a hooded cap type, wherein the annular inward protrusion 162 is arranged on an inner wall of a cap rim, the force transmission cylinder 161 is arranged in the inner middle, the end button 6 is sleeved at a barrel post at the rear end of the end limiting member 8, the annular outward protrusion 81 is arranged at an outer side of the rear end of the barrel post, and a spherical face at the front end of the force transmission cylinder 161 abuts against a groove in the middle of an inner bottom of the barrel post at the rear end.

Further, in order to facilitate arranging the pen body, it is preferred here that the pen body includes a body barrel 15 and a push rod nut 4 fitted into the body barrel 15. The push rod nut 4 is connected to the injection push rod 13 by threads and connected to the push rod sleeve 7 through the first set of ratchet pawl mechanisms. In an embodiment, an opening is defined at the front end of the body barrel 15 and a limiting shoulder is provided within the body barrel 15. The push rod nut 4 is inserted into the body barrel 15 through a front end opening until the push rod nut 4 abuts against the limiting shoulder, and a limiting protrusion 155 is arranged within the body barrel 15 to prevent the push rod nut 4 from disengaging from the body barrel 15. When the push rod nut 4 is installed, the push rod nut 4 can get over the limiting protrusion 155 through elastic deformation. In an embodiment, the push rod nut 4 may be provided with a threaded hole 41 which extends through the push rod nut 4 in the front-rear direction, and a large groove is defined in a rear section. The wall of the large groove is provided with the limiting ratchets 42 of the first set of ratchet pawl mechanisms. Correspondingly, limiting pawls 72 of the first set of ratchet pawl mechanisms are arranged at a front end of the push rod sleeve 7. Two third grooves 43 extending in the injection direction are provided externally on two sides of the large groove, and the body barrel 15 is provided with two third protrusions 154 fitting with the third grooves 43.

In an embodiment, the injection push rod 13, the push rod sleeve 7, the clutch hub 6, the dose limiting nut 5, the dose hub 14 and the body barrel 15 are arranged in sequence with a latter sleeved on a former. In order to avoid excessive axial movement of the clutch hub 6, it is preferred here that a hook 74 is arranged at the rear end of the push rod sleeve 7.

Further, in order to facilitate reading the dose, it is preferred here that a window 152 is arranged on the body barrel 15 and spirally distributed scales are provided at an outer side of the dose hub 14 to pass the window 152 in sequence when the dose hub is screwed in. For a more precise reading, it is preferred here that the body barrel 15 has a reading mark 153, preferably in the form of an arrow. Correspondingly, the dose hub 14 may be provided with a zero mark 143 corresponding to a zero point of the scales. When the reading is zero, the zero mark 143 is aligned with the reading mark 153. For ease of rotation, it is preferred here that radial splines 144 exposed to the body barrel 15 are provided at the rear end of the dose hub 14, to prevent slipping.

Further, in order to facilitate the fixation of the cartridge, it is preferred here that a vial holder 10 is further included. The vial holder is clamped at the front end of the body barrel. In an embodiment, the rear end of the vial holder 10 is inserted into the front end opening of the body barrel 15, and a notch 101 is provided at the rear end to position the angular position relative to the body barrel 15. Further, an annular mounting protrusion 102 is provided to fit with an annular mounting groove 156 within the opening of the body barrel 15, so as to position and firmly secure the vial holder 10 in the injection direction. A vial ring 12 is arranged at the rear end of the vial holder 10, the vial ring 12 is configured to abut against a vial in the vial holder 10 at the front end and abut against the pen body at the rear end. A rotatable push rod knob 11 configured to abut against a vial rubber stopper is provided at the front end of the injection push rod 13, and a threaded post 104 is provided at the front end of the vial holder 10 for attaching a needle 1. Correspondingly, a pen cap 9 is further provided. A rear end of the pen cap 9 is snap-fitted to the vial holder 10. That is, limiting connection structures 103 are respectively provided on two opposite sides of the vial holder 10, and corresponding limiting connection structures are provided at an inner side of a rear end of the pen body.

The embodiments in this specification are described in a progressive manner. Each embodiment focuses on differences from other embodiments, and for the same or similar parts among the embodiments, reference can be made to one another.

Based on the above description of the disclosed embodiments, those skilled in the art can implement or practice the present application. Various modifications to these embodiments are apparent to those skilled in the art. The general principles defined herein may be implemented in other embodiments without departing from the spirit or scope of the present application. Therefore, the present application should not be limited to the embodiments disclosed herein, but has the widest scope in accordance to the principle and the novel features disclosed herein.

## Claims

1. A multi-dose adjustable pre-filled injection pen, comprising:
a pen body;
an injection push rod;
a push rod sleeve;
a clutch hub;
an end limiting member; and
a dose hub; wherein
the injection push rod is connected to the pen body through threads having an axis extending in an injection direction,
the push rod sleeve is in a sliding fit with the injection push rod in the injection direction, which is configured to transmit a torque in a direction of screwing-in of the injection push rod,
the push rod sleeve is connected to the pen body through a first set of ratchet pawl mechanisms, which precludes the push rod sleeve from rotating, relative to the pen body, in a direction of screwing-out of the injection push rod,
the clutch hub is in a sliding fit with the push rod sleeve in the injection direction, and relative rotation between the clutch hub and the push rod sleeve is precluded,
the dose hub is rotatably connected to the pen body through threads, and is configured to be pushed to screw in by driving axially,
relative rotation between the clutch hub and the dose hub is precluded by a clutch structure which disengages in the injection direction,
a support foot elastically deformable in the injection direction is braced between the end limiting member and the dose hub,
the clutch structure is prevented from disengaging by the support foot, which results from the deformation of the support foot due to the fact that the end limiting member is subjected to a thrust in the injection direction, and
the clutch structure is configured to be pushed to disengage by rotation of the dose hub before the support foot deforms elastically.

2. The multi-dose adjustable pre-filled injection pen according to claim 1, further comprising a dose limiting nut, wherein the dose limiting nut is connected to the clutch hub by threads and in a sliding fit with the dose hub in the injection direction, relative rotation between the dose limiting nut and the dose hub is precluded, and the dose limiting nut is moved along an axial direction of the clutch hub when the dose hub is rotated relative to the clutch hub.

3. The multi-dose adjustable pre-filled injection pen according to claim 2, wherein a pawl is arranged at a tip of the support foot, and a ratchet fitting with the pawl of the support foot is arranged at an end of the dose hub, to preclude relative rotation in the direction of screwing-in;
the end limiting member is provided with a limiting flange, and the dose hub is correspondingly provided with a limiting groove enclosing the limiting flange to limit a range of movement of the limiting flange in the injection direction.

4. The multi-dose adjustable pre-filled injection pen according to claim 3, wherein a protruding disc is arranged at a rear end of the clutch hub,
a plurality of arc-shaped protrusions circumferentially evenly distributed are arranged at a front side of the protruding disc,
an annular shoulder is arranged inside a rear end of the dose hub,
arc-shaped tooth grooves circumferentially distributed and fitting with the arc-shaped protrusions are arranged at a rear side of the annular shoulder; and
the rear end of the clutch hub and the end limiting member are connected through a second set of ratchet pawl mechanisms.

5. The multi-dose adjustable pre-filled injection pen according to claim 4, further comprising an end button, wherein a circumference of the end button is fitted into the end limiting member, a force transmission cylinder extending in the injection direction is arranged in the middle of the end button, and a front end of the force transmission cylinder abuts against the end limiting member and is spherical.

6. The multi-dose adjustable pre-filled injection pen according to claim 5, wherein the pen body comprises a body barrel and a push rod nut fitted into the body barrel, wherein the push rod nut is connected to the injection push rod by threads and connected to the push rod sleeve through the first set of ratchet pawl mechanisms.

7. The multi-dose adjustable pre-filled injection pen according to claim 6, wherein the injection push rod, the push rod sleeve, the clutch hub, the dose limiting nut, the dose hub and the body barrel are arranged in sequence with the latter sleeved on the former one by one.

8. The multi-dose adjustable pre-filled injection pen according to claim 7, wherein a window is provided on the pen body, and spirally distributed scales are provided at an outer side of the dose hub to pass through the window in sequence when the dose hub is screwed in.

9. The multi-dose adjustable pre-filled injection pen according to claim 8, further comprising a vial holder clamped at a front end of the body barrel, wherein a vial ring is arranged at a rear end of the vial holder; the vial ring is configured to abut against a vial in the vial holder at a front end and abut against the pen body at a rear end,
a rotatable push rod knob configured to abut against a vial rubber stopper is provided at a front end of the injection push rod, and
a threaded post is provided at a front end of the vial holder for attaching a needle.

10. The multi-dose adjustable pre-filled injection pen according to claim 9, further comprising a pen cap, wherein a rear end of the pen cap is snap-fitted to the vial holder.
